(19) Europäisches Patentamt
European Patent Office
Office européen des brevets

(11) **EP 2 433 125 B1**

(12) **EUROPEAN PATENT SPECIFICATION**

(45) Date of publication and mention
of the grant of the patent:
**28.11.2018 Bulletin 2018/48**

(51) Int Cl.:
***G01N 33/50*** (2006.01)

(21) Application number: **10721224.3**

(22) Date of filing: **20.05.2010**

(86) International application number:
**PCT/US2010/035616**

(87) International publication number:
**WO 2010/135555 (25.11.2010 Gazette 2010/47)**

(54) **METHOD FOR DETERMINING THE CARDIO-GENERATIVE POTENTIAL OF MAMMALIAN CELLS**

VERFAHREN ZUR BESTIMMUNG DES KARDIOGENERATIVEN POTENZIALS VON SÄUGERZELLEN

PROCÉDÉ DE DÉTERMINATION DU POTENTIEL CARDIO-GÉNÉRATEUR DE CELLULES DE MAMMIFÈRES

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **20.05.2009 PCT/US2009/044751**

(43) Date of publication of application:
**28.03.2012 Bulletin 2012/13**

(73) Proprietors:
• **MAYO FOUNDATION FOR MEDICAL EDUCATION AND RESEARCH**
Rochester, MN 55905 (US)
• **Cardio3 BioSciences S.A.**
1435 Mont-Saint-Guibert (BE)

(72) Inventors:
• **GORDON-BERESFORD, Roland**
B-1310 La Hulpe (BE)
• **GAUSSIN, Vinciane**
B-1970 Wezembeek-Oppem (BE)
• **HOMSY, Christian**
B-1150 Bruxelles (BE)
• **TERZIC, Andre**
Rochester, Minnesota 55902 (US)
• **BEHFAR, Atta**
Rochester, Minnesota 55902 (US)

(74) Representative: **Crease, Devanand John et al**
**Keltie LLP**
**No.1 London Bridge**
**London SE1 9BA (GB)**

(56) References cited:
**WO-A1-2005/038454**

• **RAJASINGH JOHNSON ET AL: "STAT3-dependent mouse embryonic stem cell differentiation into cardiomyocytes analysis of molecular signaling and therapeutic efficacy of cardiomyocyte precommitted mES transplantation in a mouse model of myocardial infarction" CIRCULATION RESEARCH, vol. 101, no. 9, October 2007 (2007-10), pages 910-918, XP002592555 ISSN: 0009-7330**
• **KUCIA MAGDA ET AL: "Cells expressing early cardiac markers reside in the bone marrow and are mobilized into the peripheral blood after myocardial infarction" CIRCULATION RESEARCH, vol. 95, no. 12, 10 December 2004 (2004-12-10), pages 1191-1199, XP002592556 ISSN: 0009-7330**
• **GHOSH TUSHAR K ET AL: "Physical Interaction between TBX5 and MEF2C Is Required for Early Heart Development" MOLECULAR AND CELLULAR BIOLOGY, vol. 29, no. 8, April 2009 (2009-04), pages 2205-2218, XP002592557 ISSN: 0270-7306**

**(Cont. next page)**

• BONDUE A ET AL: "Mesp1 acts as a master regulator of multipotent cardiovascular progenitor specification" CELL STEM CELL, CELL PRESS, US LNKD-DOI:10.1016/J.STEM.2008.06.009, vol. 3, no. 1, 3 July 2008 (2008-07-03), pages 69-84, XP002576315 ISSN: 1934-5909

**Description**

**Field of the invention**

The present invention relates to the treatment of heart disease disorders through injection of mammalian cells. In particular, it relates to a method for quantitatively assessing the cardio-generative potential of mammalian cells, thereby allowing a good predictability of the success of repairing a heart in need. It also relates to a method for quantitatively assessing the modification of this cardio-generative potential and the cardiogenic potential of a treatment aiming at cellular differentiation, and a computer device comprising a processor, and a memory encoding one or more non-neural network programs coupled to the processor, wherein said programs cause the processor to perform a method, said method comprising calculating a CARPI.

**State of the art**

Cardiovascular diseases are leading cause of morbidity and mortality worldwide, despite advances in patient management. In contrast to tissues with high reparative capacity, heart tissue is vulnerable to irreparable damages. Cell-based regenerative cardiovascular medicine is now being pursued in the clinical setting to address heart disease disorders.

Recent advent of stem cell biology extends the scope of current models of practice from traditional palliation towards curative repair. Typically, clinical experience has been based on adult stem cells delivered in an unaltered state. First generation biologics are naive human stem cells, identified as readily accessible cytotypes. It has been shown that a few individuals improve on delivery of naive human stem cells. The state of the art in the field of naive cell transplantation in the heart of humans was described inter alia in the review carried by Abdel-Latif A. et al. 'Adult bone marrow-derived cells for cardiac repair: a systematic review and meta-analysis.' Arch Intern Med. (2007) 167:989-997, and citations therein.

To improve clinical outcome, second-generation stem cell therapies were developed to guide naive human stem cells towards the cardiac lineage prior to injection into the patient. In the review by Behfar et al. 'Guided stem cell cardiopoietic: Discovery and translation' J. Mol. and Cell. Cardiology (2008) 45: 523-529, the concept of using cardiac precursor cells, such as cardiopoietic cells, for heart regeneration was discussed.

Cardiopoietic cells have a unique phenotype: they are characterized by nuclear translocation of Nkx2.5 and MEF2C polypeptides, combined to the absence of detectable sarcomeric proteins. This cardiopoietic status corresponds to an intermediate cell phenotype, i.e. committed to the cardiac lineage but not yet fully differentiated. Non-detectable level of sarcomeric protein expression is a unique feature of cardiopoietic cells which distinguishes them from contractile and sarcomeric-containing cardiomyocyte-like cells derived from stem cells and described in other applications such as by Chunhui Xu (US publication no. 2005/0164382, corresponding to US patent no. 7,763,464) and Lough et al (US 2002/0061837, patent not granted).

Increased protein content of a transcription factor may not imply its subcellular localization, which could be either cytoplasmic or nuclear. Nuclear translocation of Nkx2.5 and MEF2C polypeptides is necessary for definitive cardiac lineage commitment. This is further explained in Behfar A. et al, (Derivation of a cardiopoietic population from human mesenchymal stem cells yields cardiac progeny, Nature Clinical Practice, 2006, 3:S78-S82). Although nuclear translocation may be qualitatively observed by immunocytochemistry or immunohistochemistry, techniques such as western blotting or Fluorescence Activated Cell Sorting (FACS) that look at total protein content are not suitable for quantitative assessment of the subcellular distribution of a polypeptide. The observation of subcellular distribution of a polypeptide, as described in US 2008/0019944 (US Patent no. 9,765,298), is not only qualitative but also time-consuming in the industrial perspective and operator-dependent. Thus clinical outcome, i.e. the cardio-generative potential of these "first-generation" naive stem cells and "second-generation" guided stem cells could not be readily predicted prior to injection.

Rajasingh et al., Circulation Research, vol. 101, no. 9, October 2007 describes the analysis of molecular signalling of STAT3-dependent mouse embryonic stem cell differentiation to cardiomyocytes. Rajasingh *et al.* is limited to the measurement and monitoring of the individual expression level of genes.

A method to quantitatively assess the cardio-generative potential of mammalian cells remained to be proposed.

The present invention now provides such a predictive method for determining the cardio generative potential of mammalian cells which comprises the quantitative assessment of a CARdiac generation Potential Index (CARPI) as a function of the quantification of the expression of genes of said cells. It also addresses the quantitative assessment of the modification of the cardio generative potential of mammalian cells and the cardiogenic potential of a treatment aiming at cellular differentiation.

**Definitions**

Within the frame of the present document, and unless indicated to the contrary, the terms designated below between quotes have the following definitions.

The *'cardio-generative potential'* of a cell designates the ability of this cell to succeed to generate heart cells, for instance cardiac myocytes.

*'Cardiopoietic cells'* are an intermediate cell phenotype, i.e. committed to the cardiac lineage but not yet fully differentiated. Cardiopoietic cells are characterized by nuclear translocation of Nkx2.5 and MEF2C, combined to the absence of detectable sarcomeric proteins (Behfar et al. 'Derivation of a cardiopoietic population from human mesenchymal stem yields progeny', Nature Clin. Pract., Cardiovasc. Med. (2006) 3: S78-S82). Cardiopoietic cells retain a proliferative capacity. Cardiopoietic cells can be derived from stems cells including for example, human adult mesenchymal stem cells (Terzic et al. US publication no. 2008/0019944, corresponding to US patent no. 9,765,298), mouse embryonic stem cells (Behfar et al, 'Cardiopoietic programming of embryonic stem cells for tumour-free heart repair' J Exp Med 2007 204: 405-420*)*, embryonic-like stem cells, inducible pluripotent stem cells, umbilical cord blood cells, resident cardiac stem cells and the like, or any other adapted source (provided their production implies no human embryo destruction).

A *'cocktail'* or *'cardiogenic cocktail'* designates a composition containing at least two cardiogenic substances.

A *'cardiogenic treatment'* is a treatment which improves the cardio-generative potential of a cell. Example of such treatment consists in putting said cell in contact with a cocktail. Examples of such cocktails comprise at least two substances selected in the group consisting of growth factors, cytokines, hormones and combinations thereof. Said at least two substances may be selected in the group consisting of bone morphogenetic proteins (BMP) such as BMP-1, BMP-2, BMP-5, BMP-6; epidermal growth factor (EGF); erythropoietin (EPO); fibroblast growth factors (FGF) such as FGF-1, FGF-4, FGF-5, FGF-12, FGF-13, FGF-15, FGF-20; granulocyte-colony stimulating factor (G-CSF); granulocyte-macrophage colony stimulating factor (GM-CSF); growth differentiation factor-9 (GDF-9); hepatocyte growth factor (HGF); insuline-like growth factor (IGF) such as IGF-2; myostatin (GDF-8); neurotrophins such as NT-3, NT-4, NT-1 and nerve growth factor (NGF); platelet-derived growth factor (PDGF) such as PDGF-beta, PDGF-AA, PDGF-BB; thrombopoietin (TPO); transforming growth factor alpha (TGF-$\alpha$); transforming growth factors $\beta$ (TGF-$\beta$) such as TGF-$\beta$1, TGF-$\beta$2, TGF-$\beta$3; vascular endothelial growth factor (VEGF) such as VEGF-A, VEGF-C; TNF-$\alpha$; leukemia inhibitory factor (LIF); interleukin 6 (IL-6); retinoic acid; stromal cell-derived factor-1 (C SDF-1); brain-derived neurotrophic factor (BDNF); periostin; angiotensin II; Flt3 ligand; glial-derived neurotrophic factor; heparin; insulin-like growth factor binding protein-3; insulin-like growth factor binding protein-5; interleukin-3; interleukin-8; midkine; progesterone; putrescine; stem cell factor; Wnt1; Wnt3a; Wnt5a; caspase-4; chemokine ligand 1; chemokine ligand 2; chemokine ligand 5; chemokine ligand 7; chemokine ligand 11; chemokine ligand 20; haptoglobin; lectin; cholesterol 25-hydroxylase; syntaxin-8; syntaxin-11; ceruloplasmin; complement component 1; complement component 3; integrin alpha 6; lysosomal acid lipase 1; $\beta$-2 microglobulin; ubiquitin; macrophage migration inhibitory factor; cofilin; cyclophillin A; FKBP12; NDPK; profilin 1; cystatin C; calcyclin; stanniocalcin-1; PGE-2; mpCCL2; IDO; iNOS; HLA-G5; M-CSF; angiopoietin; PIGF; MCP-1; extracellular matrix molecules; CCL2 (MCP-1); CCL3 (MIP-1$\alpha$); CCL4 (MIP-1$\beta$); CCL5 (RANTES); CCL7 (MCP-3); CCL20 (MIP-3$\alpha$); CCL26 (eotaxin-3); CX3CL1 (fractalkine); CXCL5 (ENA-78); CXCL11 (i-TAC); CXCL1 (GRO$\alpha$); CXCL2 (GRO$\beta$); CXCL8 (IL-8); CCL10 (IP-10); and combinations thereof.

A *'cocktail-guided cell'* or a *'cell guided towards cardiac differentiation'* is a cell which has been put into contact with a cocktail.

*'Differentiation'* is the process by which a less specialized cell becomes a more specialized cell.

*'Ejection fraction'* means the fraction of blood pumped out during a heartbeat. Without a qualifier, the term ejection fraction refers specifically to that of the left ventricle (left ventricular ejection fraction or LVEF).

As used in the subject specification, the singular forms *'a'*, *'an'* and *'the'* include plural aspects unless the context clearly dictates otherwise. Thus, for example, reference to *'a stem cell'* includes a single cell, as well as two or more cells; reference to *'an agent'* or *'a reagent'* includes a single agent or reagent, as well as two or more agents or reagents; reference to 'the invention' or 'an invention' includes single or multiple aspects of an invention; and so forth.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

**Summary of the invention**

The invention provides an *in vitro* method for determining the cardio-generative potential of cardiopoietic cells, wherein said cells are characterized by nuclear translocation of Nkx2.5 and MEF2C and contain no detectable sarcomeric proteins, said method comprising the assessment of a CARdiac generation Potential Index (CARPI) which is a function of the quantification of the expression of two or more genes of said cells and which is calculated as a linear average of the expression level at the RNA level of said two or more genes, wherein said genes are selected from the group consisting of Nkx2.5, Tbx5, MEF2C, GATA4, GATA6, Mesp1, FOG1 wherein the CARPI is calculated using the formula:

$$CARPI = \frac{1}{n} \sum_{i=1}^{i=n} RNA\,level_i \quad ,$$

where i represents the selected gene and n represents the total number of genes selected.

The CARPI may be a function of the quantification of messenger RNA (mRNA) levels of specific genes of said cells.

The cells may be somatic, umbilical cord blood, cardiac progenitor, and/or genetically modified cells, and wherein said cells are not germinal cells.

In some cases, the cells can belong to one individual, and a CARPI can be assessed for those cells before and after exposing the cells to any cardiogenic treatment, suitably the cardiogenic treatment comprises exposing said cells to a cocktail containing cardiogenic substances.

In a further embodiment, the cells belong to one mammal.

In another embodiment, a CARPI is assessed for cells of an individual or group of individuals *versus* another individual or group of individuals.

In a particular embodiment of a method provided herein a CARPI is measured to quantitatively assess the cardiogenic potential of a treatment.

According to one embodiment of a method provided herein, the CARPI may be put into correlation with a parameter of cardiac function.

The invention also relates to a computer device comprising a processor, and a memory encoding one or more programs coupled to the processor, wherein the one or more programs cause the processor to perform a method, said method comprising the steps of:

a) collecting the expression level at the RNA level of two of more genes of cardiopoietic cells, wherein said cells are characterized by nuclear translocation of Nkx2.5 and MEF2C and contain no detectable sarcomeric proteins, wherein said genes are selected from the group consisting of Nkx2.5, Tbx5, MEF2C, GATA4, GATA6, Mesp1, FOG1;

b) determining a CARPI calculated as a linear average of said expression levels of said two or more genes using the formula:

$$CARPI = \frac{1}{n} \sum_{i=1}^{i=n} RNA\,level_i \quad ,$$

where i represents the selected gene and n represents the total number of genes selected; and

c) displaying the CARPI.

**Brief Description of the Drawing**

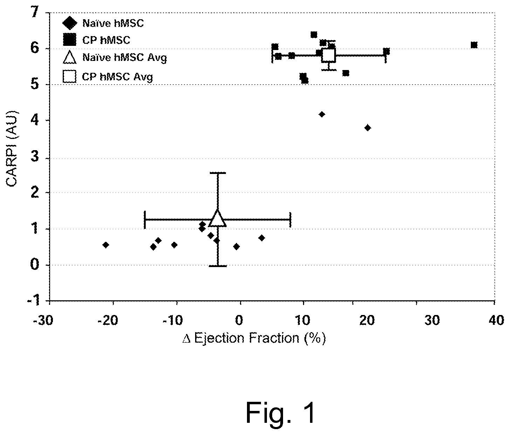

Fig. 1 shows in Y ordinate the CARPI, in arbitrary units (AU), calculated for both naive human MSC (hMSC) and cocktail guided-hMSC (CP-hMSC) on the basis of quantification of the expression of genes at the mRNA level and in X ordinate the change of LVEF ($\Delta$EF) in percent prior and after injection in mouse infarcted hearts. Black symbols represent individual data; open symbols represent averaged data (Avg).

**Detailed description of the invention**

**Example 1**

Bone marrow samples were harvested from patients undergoing coronary artery bypass for ischemic heart disease. Patients provided informed consent, as approved by competent Institutional Ethics Committees.

Mesenchymal stem cells were recruited by plating of raw bone marrow on plastic dishes, with a wash at 12h, selecting adhesive cells with identity confirmed by Fluorescence-Activated Cell Sorting (FACS) analysis using the CD34⁻/CD45⁻/CD133⁺ marker panel. Cells were further cultured and expanded at 37° C in DMEM supplemented with 5% human platelet lysate (Mayo Clinic Blood Bank, Rochester, MN).

Naive human bone marrow-derived mesenchymal stem cells were cultured in either platelet lysate or serum supplemented with a cardiogenic cocktail consisting in TGFβ-1 (2.5 ng/ml), BMP4 (5 ng/ml), FGF2 (5 ng/ml), IGF-1 (50 ng/ml), Activin-A (10 ng/ml), Cardiotrophin (1 ng/ml), α-thrombin (1 U/ml), and Cardiogenol C (100 nM) in order to derive a cardiopoietic population.

The present invention allows the quantitative assessment of the cardio-generative potential of said cardiopoietic population, by quantifying the expression of two or more genes at the RNA level. This invention obviates the problems of qualitative observations, issue of time, and operator-dependence, inherent to the observation of subcellular location of transcription factor polypeptides. One method of choice is real-time quantitative reverse transcription polymerase chain reaction (qPCR). This method gives faster results (within one day) that are operator-independent and quantified relative to a reference standard. In addition, while immunostained samples require one-by-one fluorescent microscopy evaluation, up to 48 different samples (or conditions) can be tested in duplicate by qPCR using 96-well plates.

In order to identify suitable markers for qPCR, mRNA was extracted from cardiopoietic cells that were evaluated by immunofluorescence staining.

The reference standard consisted of cells from the same batch cultured in the absence of the cardiogenic cocktail.

Genes listed in Table 1, which are representative of cardiac transcriptional activity were evaluated.

qPCR was performed using a TaqMan PCR kit with an Applied Biosystems 7,900HT Sequence Detection System (Applied Biosystems, Foster City, CA). TaqMan Gene Expression reactions were incubated in a 96-well plate and run in triplicate. The threshold cycle ($C_T$) was defined as the fractional cycle number at which fluorescence passes a fixed threshold. TaqMan $C_T$ values were converted into relative fold changes determined using the $2^{-\Delta\Delta C_T}$ method, normalized to a housekeeping gene expression, i.e. GAPDH (P/N 435,2662-0506003).

Results for treated cells were normalized to results obtained for the corresponding reference standard.

A CARPI, which is a function of the quantification of the expression of two or more genes of said cells, was calculated as a linear average of the expression at the RNA level of Nkx2.5, Tbx-5, MEF2C, GATA-4, GATA-6, MESP-1 and FOG-1 using a calculation spreadsheet (Microsoft Excel 2007®, Microsoft Corporation). The following formula was used:

$$CARPI = \frac{1}{n} \sum_{i=1}^{i=n} RNA\ level_i$$

where '$i$' represents the selected gene and '$n$' represents the total number of genes selected, with a minimum of 2. In this particular example, n=7.

The cardio-generative potential of hMSC-derived cardiopoietic cells was evaluated in nude, immunocompromised mice (Harlan, Indianapolis, IN). The protocol was approved by the competent Institutional Animal Care and Use Committee.

Myocardial infarction was performed. Following a blinded design, one month post-infarction a total of 600,000 total viable naive hMSC or 600,000 total viable hMSC-derived cardiopoietic cells, suspended in 12.5 μl of platelet lysate-free propagation medium, were injected under microscopic visualization in five epicardial sites on the anterior wall of the left ventricle (2.5 μl per injection site).

Left ventricular function and structure were serially followed by transthoracic echocardiography (Sequoia 512; Siemens, Malvern, PA and VisualSonics Inc, Toronto, Canada). Left ventricular ejection fraction (LVEF, %) was calculated as [(LVVd - LWs)/LWd] x 100, where LWd is left ventricular end-diastolic volume (μl), and LVVs is left ventricular end-systolic volume (μl).

A change of LVEF (ΔEF) was calculated as the difference between LVEF measured one month after cell injection and LVEF measured prior to cell injection.

Fig. 1 is a graph plotting the CARPI for each individual cell culture against the corresponding ΔEF for the mouse injected with the respective said individual cell culture. Naive hMSC (small black diamonds) typically demonstrated a low CARPI associated with no significant improvement in myocardial function (negative ΔEF) one month post-cell injection. It is worth noting rare batches of naive hMSCs innately possessing high CARPI value together with an innate regenerative potential. The average for all batches of naive hMSCs is shown by a large white triangle. hMSC-derived cardiopoietic cells (small black squares) typically demonstrated an elevated CARPI associated with robust increase in myocardial function (positive ΔEF). The average for all batches of hMSC-derived cardiopoietic cells is shown by a large white square. Averages are represented together with the corresponding 95% confidence interval.

Thus, the inventors demonstrate that there is a positive correlation between an elevated CARPI of the cells to be injected and the change in ejection fraction after injection in the infarcted heart. Thus, the CARPI is a predictive index of cardio-generative potential.

Table 1

| Applied Biosystems Assay ID | Gene name | Gene symbol |
|---|---|---|
| Hs00231763_m1 | -Homeobox transcription factor or<br>-NK2 transcription factor related, locus 5 | Nkx2.5 or NKX2-5 or NKX2.5 |
| Hs00171403_m1 | -Zinc finger cardiac transcription factor or<br>-GATA binding protein 4 | GATA-4 or, GATA4 (AB) |
| Hs00231149_m1 | -Myocyte enhancer factor 2C | MEF2c or MEF2C |
| Hs00361155_m1 | -T-box transcription factor or<br>-T-box 5 | Tbx5 or TBX5 |
| Hs00542350_m1 | -GATA co-factor ("Friend of GATA") or<br>-zinc finger protein, multitype 1 | FOG 1 of FOG-1 or FOG1 |
| Hs00251489_m1 | -Helix-loop-helix transcription factor<br>-Mesoderm posterior 1 homolog (mouse) (AB) | Mesp1 or MESP1 |
| Hs00232018_m1 | -GATA binding protein 6 (AB) | GATA-6 or GATA6 |
| Hs00911699_m1 | -Kinase insert domain receptor (a type III receptor tyrosine kinase) | Flk-1, or FLK1 or KDR |

## Example 2

Similar results have been observed by treating stem cells with a cocktail containing recombinant TGFβ-1 (2.5 ng/ml), BMP4 (5 ng/ml), Activin-A (5 ng/ml), FGF-2 (10 ng/ml), α-thrombin (1 U/ml), IGF-1 (50 ng/ml), Cardiotrophin (1 ng/ml) and Cardiogenol C (100 nM) used in a combinatorial fashion.

## Example 3

Similar results have been observed by treating stem cells with a cocktail containing recombinant TGF-β1 (2.5 ng/ml), BMP-4 (5 ng/ml), Activin-A (5 ng/ml), FGF-2 (10 ng/ml), α-thrombin (1 U/ml), IGF-1 (50 ng/ml), IL-6 (100 ng/ml) and retinoic acid (1 μM) used in a combinatorial fashion.

## Claims

1. An *in vitro* method for determining the cardio-generative potential of cardiopoietic cells, wherein said cells are **characterized by** nuclear translocation of Nkx2.5 and MEF2C and contain no detectable sarcomeric proteins, said method comprising the assessment of a CARdiac generation Potential Index (CARPI) which is a function of the quantification of the expression of two of more genes of said cells and which is calculated as a linear average of the expression level at the RNA level of said two or more genes, wherein said genes are selected from the group consisting of Nkx2.5, Tbx5, MEF2C, GATA4, GATA6, Mesp1, FOG1 wherein the CARPI is calculated using the formula:

$$CARPI = \frac{1}{n} \sum_{i=1}^{i=n} RNA\ level_i$$

,

where i represents the selected gene and n represents the total number of genes selected.

2. The method according to claim 1, wherein said cells are selected from the group consisting of somatic, umbilical cord blood, cardiac progenitors cells and any combination thereof.

3. The method according to claim 1 or claim 2, wherein said cells are genetically modified, and wherein said cells are not germinal or embryonic cells.

4. The method according to any of the preceding claims, wherein the CARPI is assessed for said cells before and after any cardiogenic treatment.

5. The method according to claim 4, wherein the cardiogenic treatment comprises exposing said cells to a cocktail containing cardiogenic substances.

6. The method according to any of the preceding claims, wherein said cells belong to one mammal.

7. The method according to any of claims 1 to 6, wherein cells are from a mammal or group of mammals and the CARPI is assessed and compared to the CARPI of cells belonging to another mammal or group of mammals.

8. The method according to any of the preceding claims, wherein the CARPI is measured to quantitatively assess the cardiogenic potential of a treatment.

9. The method according to any of the preceding claims, wherein the CARPI is put into correlation with a parameter of cardiac function.

10. A computer device comprising a processor, and a memory encoding one or more non-neural network programs coupled to the processor, wherein said programs cause the processor to perform a method, comprising the steps of:

a) collecting the expression level at the RNA level of two of more genes of cardiopoietic cells, wherein said cells are **characterized by** nuclear translocation of Nkx2.5 and MEF2C and contain no detectable sarcomeric proteins, wherein said genes are selected from the group consisting of Nkx2.5, Tbx5, MEF2C, GATA4, GATA6, Mesp1, FOG1;
b) determining a CARPI calculated as a linear average of said expression levels of said two or more genes using the formula:

$$CARPI = \frac{1}{n} \sum_{i=1}^{i=n} RNA\ level_i$$
,

where i represents the selected gene and n represents the total number of genes selected; and
c) displaying the CARPI.

**Patentansprüche**

1. Ein In-vitro-Verfahren zur Bestimmung des kardiogenerativen Potenzials von kardiopoetischen Zellen, wobei die Zellen durch eine Kerntranslokation von Nkx2.5 und MEF2C gekennzeichnet sind und keine nachweisbaren sarkomerischen Proteine enthalten, wobei das Verfahren die Untersuchung eines CARdiac Generation Potential Index (CARPI) beinhaltet, der eine Funktion der Quantifizierung der Expression von zwei oder mehr Genen der Zellen ist und der als ein linearer Mittelwert des Expressionslevels auf RNA-Ebene der zwei oder mehr Gene berechnet wird, wobei die Gene aus der Gruppe ausgewählt sind, die aus Nkx2.5, Tbx5, MEF2C, GATA4, GATA6, Mesp1, FOG1 besteht, wobei der CARPI unter Verwendung der folgenden Formel berechnet wird:

$$CARPI = \frac{1}{n} \sum_{i=1}^{i=n} RNA\ level_i$$
,

RNA level$_i$ = RNA-Level$_i$

wobei i für das ausgewählte Gen steht und n für die Gesamtzahl der ausgewählten Gene steht.

**2.** Verfahren gemäß Anspruch 1, wobei die Zellen aus der Gruppe ausgewählt sind, die aus somatischen Zellen, Nabelschnurblutzellen, Herzvorläuferzellen und jeder beliebigen Kombination davon besteht.

**3.** Verfahren gemäß Anspruch 1 oder Anspruch 2, wobei die Zellen genetisch modifiziert sind und wobei die Zellen keine Keimbahn- oder embryonalen Zellen sind.

**4.** Verfahren gemäß einem der vorhergehenden Ansprüche, wobei der CARPI vor und nach einer kardiogenen Behandlung auf die Zellen untersucht wird.

**5.** Verfahren gemäß Anspruch 4, wobei die kardiogene Behandlung das Exponieren der Zellen gegenüber einem Cocktail, der kardiogene Substanzen enthält, beinhaltet.

**6.** Verfahren gemäß einem der vorhergehenden Ansprüche, wobei die Zellen zu einem Säuger gehören.

**7.** Verfahren gemäß einem der Ansprüche 1 bis 6, wobei die Zellen von einem Säuger oder einer Gruppe von Säugern sind und der CARPI untersucht und mit dem CARPI von Zellen, die zu einem anderen Säuger oder einer anderen Säugergruppe gehören, verglichen wird.

**8.** Verfahren gemäß einem der vorhergehenden Ansprüche, wobei der CARPI gemessen wird, um das kardiogene Potenzial einer Behandlung zu untersuchen.

**9.** Verfahren gemäß einem der vorhergehenden Ansprüche, wobei der CARPI in Korrelation mit einem Parameter der Herzfunktion gebracht wird.

**10.** Eine Computervorrichtung, die einen Prozessor und einen Speicher beinhaltet, der ein oder mehrere nicht neurale, mit dem Prozessor gekoppelte Netzwerkprogramme kodiert, wobei die Programme den Prozessor dazu veranlassen, ein Verfahren durchzuführen, das die folgenden Schritte beinhaltet:

a) Erfassen des Expressionslevels auf RNA-Ebene von zwei oder mehr Genen von kardiopoetischen Zellen, wobei die Zellen durch Kerntranslokation von Nkx2.5 und MEF2C gekennzeichnet sind und keine nachweisbaren sarkomerischen Proteine enthalten, wobei die Gene aus der Gruppe ausgewählt sind, die aus Nkx2.5, Tbx5, MEF2C, GATA4, GATA6, Mesp1, FOG1 besteht;
b) Bestimmen eines CARPI, der als ein linearer Mittelwert der Expressionslevels der zwei oder mehr Gene unter Verwendung der folgenden Formel berechnet wird:

$$CARPI = \frac{1}{n} \sum_{i=1}^{i=n} RNA\ level_i\ ,$$

RNA level$_i$ = RNA-Level$_i$
wobei i für das ausgewählte Gen steht und n für die Gesamtzahl der ausgewählten Gene steht; und
c) Anzeigen des CARPI.

**Revendications**

**1.** Procédé de détermination *in vitro* du potentiel de régénération cardiaque de cellules cardiopoïétiques, où lesdites cellules sont **caractérisées par** une translocation nucléaire de Nkx2.5 et de MEF2C et ne contiennent aucune protéine sarcomérique détectable, ledit procédé comprenant l'évaluation d'un indice du potentiel de régénération cardiaque (CARPI, pour *CARdiac generation Potential Index*) qui est fonction de la quantification de l'expression de deux ou plusieurs gènes dans lesdites cellules et qui est calculé en tant que la moyenne linéaire du taux d'expression au niveau de l'ARN desdits deux ou plusieurs gènes, lesdits gènes étant sélectionnés dans le groupe consistant en Nkx2.5, Tbx5, MEF2C, GATA4, GATA6, Mesp1 et FOG1 et le CARPI étant calculé en utilisant la formule suivante :

$$CARPI = \frac{1}{n} \sum_{i=1}^{i=n} RNA\ level_i$$

,

RNA level$_i$ = Taux d'expression de l'ARN$_i$
où i représente le gène sélectionné et n représente le nombre total de gènes sélectionnés.

2. Procédé selon la revendication 1, où lesdites cellules sont sélectionnées dans le groupe consistant en des cellules somatiques, des cellules du sang du cordon, des cellules souches cardiaques et toute combinaison de celles-ci.

3. Procédé selon la revendication 1 ou la revendication 2, où lesdites cellules sont génétiquement modifiées et où lesdites cellules ne sont pas des cellules germinales ou embryonnaires.

4. Procédé selon l'une quelconque des revendications précédentes, où le CARPI est évalué pour lesdites cellules avant et après toute thérapie cardiogénique.

5. Procédé selon la revendication 4, où la thérapie cardiogénique comprend l'exposition desdites cellules à un cocktail contenant des substances cardiogéniques.

6. Procédé selon l'une quelconque des revendications précédentes, où lesdites cellules appartiennent à un mammifère.

7. Procédé selon l'une quelconque des revendications 1 à 6, où les cellules sont obtenues chez un mammifère ou un groupe de mammifères et où le CARPI est évalué et comparé au CARPI de cellules appartenant à un autre mammifère ou groupe de mammifères.

8. Procédé selon l'une quelconque des revendications précédentes, où le CARPI est mesuré pour évaluer quantitativement le potentiel cardiogénique d'une thérapie.

9. Procédé selon l'une quelconque des revendications précédentes, où le CARPI est mis en corrélation avec un paramètre de la fonction cardiaque.

10. Dispositif informatisé comprenant un processeur et une mémoire codant pour un ou plusieurs programmes qui ne reposent pas sur des réseaux de neurones artificiels couplés au processeur, où lesdits programmes font que le processeur exécute un procédé comprenant les étapes consistant à :

a) analyser le taux d'expression au niveau de l'ARN de deux ou plusieurs gènes de cellules cardiopoïétiques, où lesdites cellules sont **caractérisées par** une translocation nucléaire de Nkx2.5 et de MEF2C et ne contiennent aucune protéine sarcomérique détectable, où lesdits gènes sont sélectionnés dans le groupe consistant en Nkx2.5, Tbx5, MEF2C, GATA4, GATA6, Mesp1 et FOG1 ;
b) déterminer un CARPI qui est calculé en tant que la moyenne linéaire desdits taux d'expression desdits deux ou plusieurs gènes en utilisant la formule suivante :

$$CARPI = \frac{1}{n} \sum_{i=1}^{i=n} RNA\ level_i$$

,

RNA level$_i$ = Taux d'expression de l'ARN$_i$
où i représente le gène sélectionné et n représente le nombre total de gènes sélectionnés ; et
c) afficher le CARPI.

Fig. 1

## REFERENCES CITED IN THE DESCRIPTION

**Patent documents cited in the description**

- US 20050164382 A, Chunhui Xu
- US 7763464 B
- US 20020061837 A, Lough

- US 20080019944 A
- US 9765298 B

**Non-patent literature cited in the description**

- **ABDEL-LATIF A. et al.** Adult bone marrow-derived cells for cardiac repair: a systematic review and meta-analysis. *Arch Intern Med.,* 2007, vol. 167, 989-997
- **BEHFAR et al.** Guided stem cell cardiopoietic: Discovery and translation. *J. Mol. and Cell. Cardiology,* 2008, vol. 45, 523-529
- **BEHFAR A. et al.** Derivation of a cardiopoietic population from human mesenchymal stem cells yields cardiac progeny. *Nature Clinical Practice,* 2006, vol. 3, S78-S82

- **RAJASINGH et al.** *Circulation Research,* October 2007, vol. 101 (9
- **BEHFAR et al.** Derivation of a cardiopoietic population from human mesenchymal stem yields progeny. *Nature Clin. Pract., Cardiovasc. Med.,* 2006, vol. 3, S78-S82
- **BEHFAR et al.** Cardiopoietic programming of embryonic stem cells for tumour-free heart repair. *J Exp Med,* 2007, vol. 204, 405-420
- *GAPDH,* vol. 435, 2662-0506003